# EUROPEAN PATENT APPLICATION

(11) **EP 4 595 744 A1**
(43) Date of publication of application: **06.08.2025**
(21) Application number: 23871463.8
(22) Date of filing: 03.08.2023
(51) Int. Cl.: A01K 23/00, A61F 13/49, A61F 13/494

(54) **ABSORBENT ARTICLE FOR PETS**

(30) Priority: 28.09.2022 JP 2022154755
(71) Applicant: DAIO PAPER CORPORATION, Shikokuchuo-shi Ehime 799-0492 (JP)
(72) Inventor: HONJO, Ryota, Shikokuchuo-shi, Ehime 799-0113 (JP)
(74) Representative: Epping - Hermann - Fischer
(86) International application number: PCT/JP2023/028424
(87) International publication number: WO 2024/070221

(57) **Abstract**

This absorbent article for pets includes a top sheet, a waterproof sheet, an absorber, a rising gather, and a waist sheet. The distance between a ventral-side edge of the waist sheet and a ventral-side edge of the absorbent article is 0 to 5 mm. The distance between a ventral-side edge of the absorber and the ventral-side edge of the absorbent article is 15 to 35 mm. The rising gather has both ends thereof in the front-back direction fixed to the top sheet. The distance between a fixation starting end on the ventral side of the rising gather and the ventral-side edge of the absorber is 0 to 5 mm. The waist sheet includes: a first region that overlaps a portion of the absorber on the ventral side; and a second region that extends beyond the first region toward the ventral side and toward the outer side in the width direction and that does not overlap the absorber. The second region is elastically stretchable in the width direction, but the first region is not elastically stretchable in the width direction.

## Description

### TECHNICAL FIELD

The present invention relates to an absorbent article for pets.

### BACKGROUND ART

In recent years, an absorbent article, such as a disposable diaper for pets such as dogs and cats, has been proposed. In many cases, the absorbent article for pets is based on a human diaper, and adjustments made to fit the body shape of pets have been discussed. Specifically, in the case of a male dog, where a urethral opening is located at the center portion of the body, the absorbent article for pets is designed to cover the urethral opening when the male dog wears it, thereby preventing urine from leaking out.

For example, the patent document 1 discloses a diaper for a male pet including a diaper body which includes a back pad portion that is brought into contact with the vicinity of a back of the male pet, a crotch pad portion that is brought into contact with the vicinity of a crotch of the male pet, and a belly pad portion that is brought into contact with the vicinity of a belly of the male pet, and has a urine absorbing body in the belly pad portion thereof. In the belly pad portion, a fixing tape is provided at a position corresponding to a waist part of the male pet when the diaper is worn so as to extend to the outside of the diaper body on both sides of the belly pad portion, and a urine capture region is formed so as to extend in the direction opposite to the crotch portion from the fixing tapes, and has an absorber that is set at a position and size so as to cover the male pet's urethral opening and its vicinity when the diaper is worn.

### RELATED-ART DOCUMENTS

### PATENT DOCUMENTS

Patent Document 1: Japanese Patent No. 4490080

### SUMMARY OF THE INVENTION

### PROBLEM TO BE SOLVED BY THE INVENTION

However, in the conventional absorbent article for pets described in Patent Document 1, since the space formed between the male pet's urethral opening and the absorber is narrow, the absorber may come into close contact with the male pet's urethral opening, or the urethral opening may be pushed up by the absorber toward its ventral side, and thus there is a possibility that the absorber cannot sufficiently capture urine, resulting in leakage of the urine.

In view of the above, an object of one embodiment of the present invention is to provide an absorbent article for pets capable of suppressing leakage of urine to the outside.

### MEANS TO SOLVE THE PROBLEM

One aspect of the present invention is an absorbent article for pets, including a front-back direction, a width direction orthogonal to the front-back direction, a dorsal-side region, and a ventral-side region, wherein the absorbent article includes: a top sheet having liquid permeability, a waterproof sheet having liquid impermeability, an absorber provided between the top sheet and the waterproof sheet, a rising gather provided on both sides of the absorbent article in the width direction over the entire front-back direction, the rising gather rising toward a skin side, and a belt-shaped waist sheet extending in the width direction along a ventral-side edge of the absorbent article; wherein a distance between a ventral-side edge of the waist sheet and the ventral-side edge of the absorbent article is 0 mm to 5 mm; wherein a distance between a ventral-side edge of the absorber and the ventral-side edge of the absorbent article is 15 mm to 35 mm; wherein the rising gather has both ends thereof in the front-back direction fixed to the top sheet; wherein a distance between a fixation starting end of the rising gather on a ventral side thereof and the ventral-side edge of the absorber is 0 mm to 5 mm; wherein the waist sheet includes: a first region overlapping a portion of the absorber on the ventral side thereof, and a second region extending beyond the first region toward a ventral side and toward an outer side in the width direction, the second region not overlapping the absorber; and wherein the second region is elastically stretchable in the width direction, and the first region is not elastically stretchable in the width direction.

### EFFECTS OF THE INVENTION

According to one embodiment of the absorbent article for pets of the present invention, leakage of urine to the outside can be suppressed.

### BRIEF DESCRIPTION OF THE DRAWINGS

FIG. 1 is a plan view of the absorbent article according to an embodiment as viewed from a skin side thereof, in which the absorbent article is in an unfolded state.
FIG. 2 is a view showing an arrangement of an adhesive layer in FIG. 1.
FIG. 3 is a cross-sectional view taken through line I-I of FIG. 1.
FIG. 4 is a cross-sectional view taken through line II-II of FIG. 2.
FIG. 5 is a perspective view of an absorbent article according to an embodiment.
FIG. 6 is a schematic view showing a state of an absorbent article according to an embodiment when worn on a pet.

### MODE FOR CARRYING OUT THE INVENTION

Hereinafter, embodiments of the present invention will be described with reference to the drawings. Note that in the drawings, unless otherwise specified, the same or corresponding components are denoted by the same reference numerals, and the description thereof may be omitted. In the present specification, a front-back direction of an absorbent article 1 is referred to as D1, and a width direction of the absorbent article 1 orthogonal to the front-back direction is referred to as D2. In the present specification, the front-back direction D1 is also used to indicate a front-back direction of the absorber 4, and the width direction D2 is also used to indicate a width direction of the absorber 4. Note that the front-back direction corresponds to a front-back direction of a pet. In the present specification, a front side of the absorbent article 1 and the absorber 4 in the front-back direction is referred to as a ventral side or front side, and a back side thereof in the front-back direction is referred to as a dorsal side or back side. In addition, a surface of the absorbent article 1 facing the pet is referred to as a skin-side surface, and a surface of the absorbent article 1 opposite to the skin surface is referred to as a non-skin-side surface.

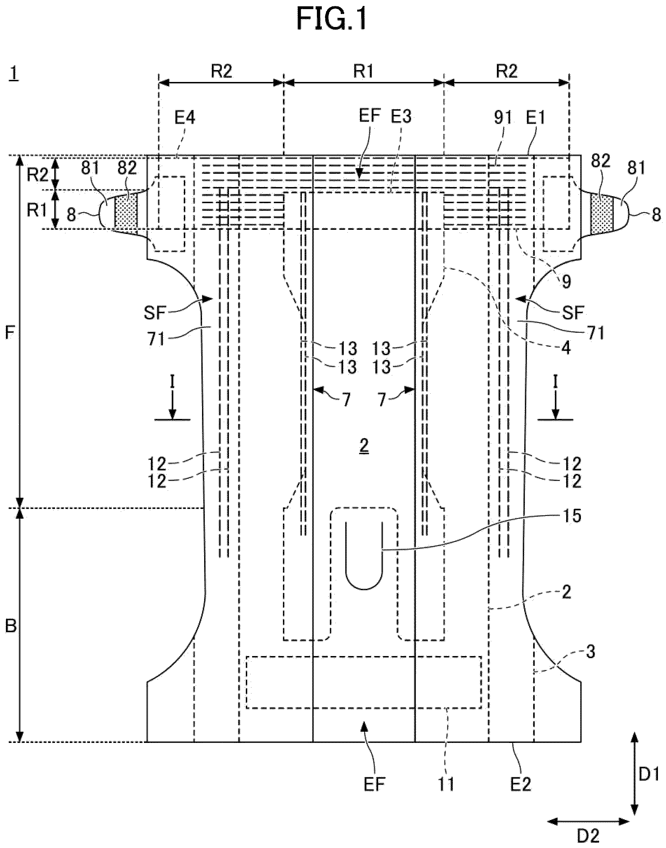

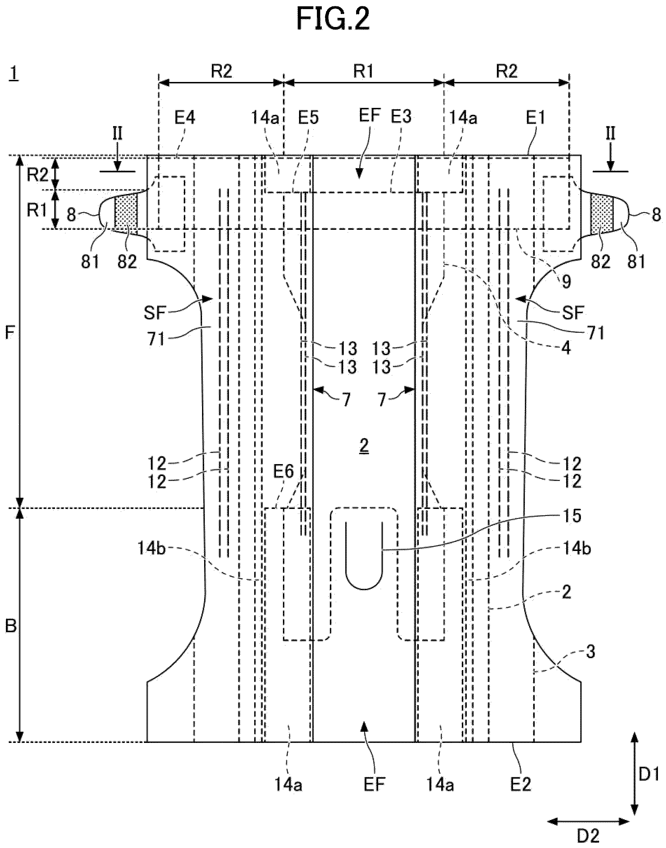

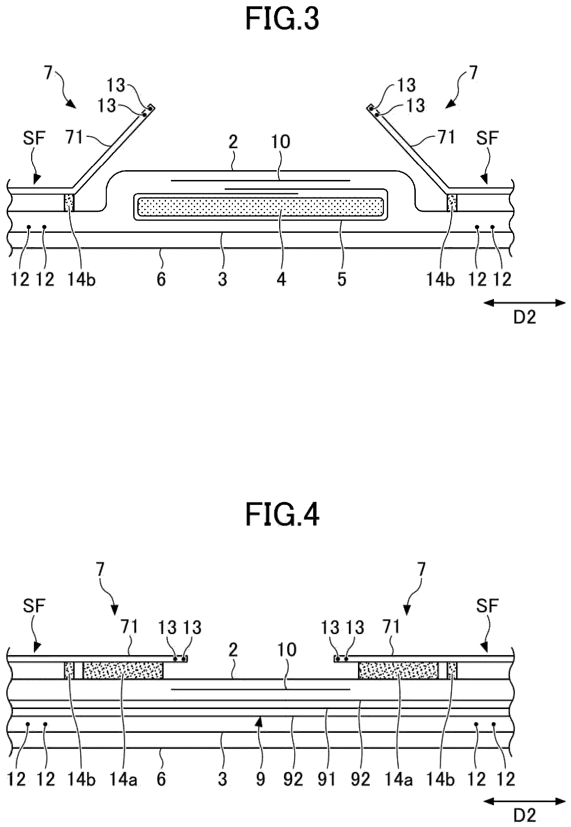

FIG. 1 is a plan view of the absorbent article according to an embodiment as viewed from the skin side thereof, in which the absorbent article is in an unfolded state, FIG. 2 is a view showing an arrangement of an adhesive layer in FIG. 1, FIG. 3 is a cross-sectional view taken through line I-I of FIG. 1, and FIG. 4 is a cross-sectional view taken through line II-II of FIG. 2.

As shown in FIG. 1, the absorbent article 1 for pets has a ventral-side region F and a dorsal-side region B. The ventral-side region F corresponds to an abdomen of the pet when the absorbent article 1 is worn, and the dorsal-side region B corresponds to a back of the pet when the absorbent article 1 is worn. The rear end of the ventral-side region F may be located, for example, 150 mm to 250 mm behind the front end of the absorbent article 1. That is, the front end of the dorsal-side region B may be located, for example, 75 mm to 300 mm in front of the rear end of the absorbent article 1.

As shown in FIGS. 1 to 3, the absorbent article 1 has a top sheet 2 having liquid permeability, a waterproof sheet 3 having liquid impermeability, an absorber 4 provided between the top sheet 2 and the waterproof sheet 3, a rising gather 7 provided on both sides in the width direction D2 over the entire front-back direction D1 and rising toward the skin side, and a belt-shaped waist sheet 9 extending in the width direction D2 along the ventral-side edge of the absorbent article 1.

The absorber 4 is provided so as to be included in the ventral-side region F and the dorsal-side region B. The absorber 4 may be wrapped by an encapsulating sheet 5 made of crepe paper, nonwoven fabric, or the like for maintaining a shape thereof and the like. The absorbent article 1 may be provided with a back sheet 6 on the non-skin side of the waterproof sheet 3. The absorbent article 1 has, on both sides thereof, a side flap SF composed of a waterproof sheet 3, a back sheet 6 and a gather sheet 71. In addition, the absorbent article 1 may have an end flap EF formed by extending the back sheet 6, the waterproof sheet 3, and the top sheet 2 in the front-back direction, without the presence of the absorber 4, and connecting tapes 8, 8 provided on both sides of the ventral-side region F in the width direction D2, respectively. When the absorbent article 1 is to be attached to a pet, the ventral-side region F and the dorsal-side region B of the absorbent article 1 are respectively applied along the pet's body, and then the connecting tapes 8, 8 can be brought to the dorsal side and engaged with a front target tape 11 provided on the outer side of the back sheet 6.

The absorbent article 1 has, as a whole, an elongated shape having a predetermined length in the front-back direction D1 and a predetermined width in the width direction D2 orthogonal to the front-back direction. The length of the absorbent article 1 in the front-back direction D1 is preferably 300 mm to 600 mm, and more preferably 340 mm to 540 mm. The length of the absorbent article 1 in the width direction D2 at the center of the length in the front-back direction D1 is preferably 160 mm to 400 mm, and more preferably 170 mm to 340 mm. Note that the term "length" herein refers to the length of the absorbent article 1 in an unfolded state. Since the length of the absorbent article 1 in the front-back direction D1 is 300 mm to 600 mm, and the length of the absorbent article 1 in the width direction D2, at the center of the length thereof in the front-back direction D1, is 160 mm to 400 mm, the absorbent article 1 can be fitted around the legs and waist of a pet when the absorbent article 1 is worn on the pet.

The dorsal-side region B of the absorbent article 1 is provided with a cut part 15 for forming a tail passage hole through which the tail passes. The cut part 15 may be a cutting line penetrating in a thickness direction of the absorbent article 1, or may be a sewing machine line intermittently penetrating in the thickness direction of the absorbent article 1. The cut part 15 may be provided in a U-shape.

The top sheet 2 is a liquid-permeable sheet that rapidly permeates an excretory liquid such as the urine. As the top sheet 2, a porous or non-porous nonwoven fabric, a porous plastic sheet, or the like is preferably used. As constituent fibers of the nonwoven fabric, for example, synthetic fibers (including single component fibers as well as composite fibers, such as core sheaths) such as olefin-based synthetic fibers (e.g., polyethylene and polypropylene), polyester-based synthetic fibers, and polyamide-based synthetic fibers; regenerated fibers such as rayon and cuprammonium rayon; and natural fibers such as cotton, etc., can be selected without a particular limitation, and can be used by mixing them. To enhance the flexibility of the nonwoven fabric, crimped fibers are preferably used as its constituent fibers. In addition, the constituent fibers of the nonwoven fabric may be hydrophilic fibers (including hydrophilic fibers rendered hydrophilic by a hydrophilizing agent), and may be hydrophobic fibers or water-repellent fibers (including water-repellent fibers rendered water repellent by a water-repellent agent) .

In addition, the nonwoven fabric is generally classified into, depending on a length of fiber, a sheet forming method, a fiber bonding method, and a lamination structure, a short fiber nonwoven fabric, a long fiber nonwoven fabric, a spun-bonded nonwoven fabric, a melt-blown nonwoven fabric, a spunlace nonwoven fabric, a thermal-bonded (through-air bonded) nonwoven fabric, a needle punch nonwoven fabric, a point bond nonwoven fabric, a laminated nonwoven fabric (an SSS (spun-bonded + spun-bonded + spun-bonded) nonwoven fabric, etc., in which same or similar nonwoven fabric layers are laminated, as well as an SMS (spun-bonded + melt-blown + spun-bonded) nonwoven fabric, an SMMS (spun-bonded + melt-blown + melt-blown + spun-bonded) nonwoven fabric, etc., in which different nonwoven fabric layers are laminated and a melt-blown layer is sandwiched between spun-bonded layers), etc., and any of these nonwoven fabrics can be used. Preferably, the laminated nonwoven fabric is manufactured as an integral nonwoven fabric including all layers, and the fiber bonding process is carried out across all layers. As a result, the texture of the absorbent article 1 can be improved, and thus a skin irritation can be prevented when the absorbent article 1 is brought into contact with the pet's skin. Note that the laminated nonwoven fabric may be a plurality of nonwoven fabrics manufactured separately and bonded together by bonding means such as a hot-melt adhesive.

The top sheet 2 extends from one end to the other end of the absorbent article 1 in the front-back direction D1, extends laterally beyond the absorber 4 in the width direction D2, and is bonded to the waterproof sheet 3. As another embodiment, a length of the top sheet 2 in the width direction D2 can be made shorter than a length of the absorber 4 in the width direction D2 as necessary, for example, when a starting point of the rising gather 7 described later is, compared to the side edge of the absorber 4, closer to the center portion of the absorber 4 in the width direction D2.

The waterproof sheet 3 is an impermeable sheet that does not allow excretory liquid to pass through. As the waterproof sheet 3, for example, a microporous sheet, which is obtained by kneading an inorganic filler in an olefinic resin such as polyethylene or polypropylene, molding the sheet, and stretching it uniaxially or biaxially, can be suitably used. Further, as the waterproof sheet 3, a nonwoven fabric may be used as a base material with enhanced waterproofness. Preferably, the waterproof sheet 3 is the same as the absorber 4 in the front-back direction D1 and the width direction D2, or extends more widely beyond the absorber 4.

The absorber 4 is a portion that absorbs and holds excretory liquid, and can be formed by an aggregate of fibers. As the fiber aggregate, not only the aggregate obtained by spinning short fibers such as cottony pulp, synthetic fibers, natural fibers using softwood or hardwood, but also a filament aggregate obtained by opening a synthetic fiber tow (fiber bundle) such as cellulose acetate as necessary can be used. As a basis weight of a fabric, in the case of spinning cottony pulp or short fibers, it can be about 100 to 300 g/m², for example, and in the case of a filament aggregate, it can be about 30 to 120 g/m², for example. In the case of a synthetic fiber, a fineness is preferably, for example, 1 to 16 dtex, more preferably 1 to 10 dtex, and more preferably 1 to 5 dtex. The absorber 4 may contain, in part or in whole, highly absorbent polymer particles. Highly absorbent polymer particles include powder in addition to particles. Highly absorbent polymer particles used in this type of absorbent article can be used as they are. For example, starch-based, cellulose-based or synthetic polymer-based materials can be used as materials for highly absorbent polymer particles. In addition, the planar view shape of the absorber 4 may be a shape in which its center portion in the front-back direction D1 is furrowed or gathered. In other words, the length of the absorber 4 in the width direction D2 may gradually decrease toward the center thereof in the front-back direction D1.

The back sheet 6 covers the entire non-skin side of the waterproof sheet 3, giving the outer surface of the absorbent article 1 a cloth-like appearance. A nonwoven fabric is used as the back sheet 6. As the constituent fibers of the nonwoven fabric, for example, synthetic fibers (including single component fibers as well as composite fibers, such as core sheaths) such as olefin-based synthetic fibers (e.g., polyethylene and polypropylene), polyester-based synthetic fibers, and polyamide-based synthetic fibers; regenerated fibers such as rayon and cuprammonium rayon; and natural fibers such as cotton, etc., can be selected without a particular limitation, and can be used by mixing them. Specifically, a spun-bonded nonwoven fabric, a melt-blown nonwoven fabric, a spunlace nonwoven fabric, a thermal-bonded (through-air bonded) nonwoven fabric, a needle punch nonwoven fabric, a point bond nonwoven fabric, a laminated nonwoven fabric (an SSS (spun-bonded + spun-bonded + spun-bonded) nonwoven fabric, etc., in which same or similar nonwoven fabric layers are laminated), etc., may be used as the nonwoven fabric. The basis weight of the fabric of the back sheet 6 is preferably 10 to 50 g/m² and more preferably 15 to 30 g/m².

The rising gather 7 has a function of preventing excrement that travels on the top sheet 2 and moves in the lateral direction, thereby preventing lateral leakage. Both ends of the rising gather 7 in the front-back direction D1 are fixed to the top sheet 2. Specifically, as shown in FIG. 2 and FIG. 4, adhesive layers 14a are provided between both ends of the rising gather 7 in the front-back direction D1 and the top sheet 2. Further, regarding the rising gather 7, its outer side in the width direction D2 is fixed, over its entire length in the front-back direction D1, to the top sheet 2. Specifically, as shown in FIG. 2 to FIG. 4, an adhesive layer 14b is provided between the outer side of the rising gather 7 in the width direction D2 and the top sheet 2 over the entire length of the rising gather 7 in the front-back direction D1.

Hot-melt adhesive is used as the adhesive constituting the adhesive layers 14a, 14b. In this embodiment, the adhesive layers 14a, 14b are used as fixing means, but any fixing means such as fusion or crimping can be used.

As shown in FIG. 3, the rising gather 7 includes a gather sheet 71 and a gather elastic member 13, and has a standing end fixed near a side edge of the absorber 4 by the adhesive layer 14b. The front end of the gather sheet 71 is folded doubly along the front-back direction D1 of the absorbent article 1, and two gather elastic members 13, 13 are arranged at the inner side of the folded part. The rising gather 7 can stand by using the contraction force of the gather elastic members 13, 13. When the rising gather 7 stands, the rising gather 7 can adhere to the body surface of the pet. Note that in the illustrated example, the number of gather elastic members 13 is two, but it may be one. On the other hand, as shown in FIG. 4, both ends of the rising gather 7 in the front-back direction D1 do not stand up, since they are fixed by the adhesive layer 14a.

As the gather elastic member 13, generally-used materials, such as styrene-based rubber, olefin-based rubber, urethane-based rubber, ester-based rubber, polyurethane, polyethylene, polystyrene, styrene-butadiene, silicone, polyester, etc., can be used.

As the gather sheet 71, a nonwoven fabric obtained by a suitable processing method such as a spun-bonding method, a through-air-bonding method, a melt-blowing method, a needle punch method, etc., can be used. Particularly for the gather sheet 71 constituting the side flap SF, it is preferable to use a nonwoven fabric excellent in air permeability with a reduced basis weight to eliminate a clumping feeling and prevent unevenness. The material fibers constituting the gather sheet 71 can be synthetic fibers such as olefin-based synthetic fibers (e.g., polyethylene and polypropylene), polyester-based synthetic fibers, and polyamide-based synthetic fibers; regenerated fibers such as rayon and cuprammonium rayon; and natural fibers such as cotton, etc.

In the side flap SF, a plurality of leg-opening elastic members 12, 12 are arranged in the front-back direction D1 of the absorbent article 1, and in the illustrated example, two leg-opening elastic members are provided, and a planar gather is formed. When the absorbent article 1 is worn, this planar gather holds the absorbent article 1 along the leg circumference, thereby improving the fit and preventing the absorbent article 1 from shifting. As the leg-opening elastic member 12, the same material as the gather elastic member 13 can be used. As shown in FIG. 2, the leg-opening elastic members 12, 12 are, in the front-back direction D1, preferably disposed at least between the fixation starting end E5 of the rising gather 7 on the ventral side and the fixation starting end E6 of the rising gather 7 on the dorsal side. As a result, since the planar gather is formed at the position in the front-back direction D1 of the absorbent article 1 where the rising gather 7 is included, the elastic stretching and contracting force of the rising gather 7 can be increased, and the urine capture space can be easily formed. Further, since the absorbent article 1 can be held along the leg circumference to improve the fit, leakage of urine into the dorsal-side region B can be suppressed, and the area of the absorber 4 in the dorsal-side region B can be reduced. Thus, the manufacturing cost of the absorbent article 1 can be reduced.

The connecting tape 8 has a tape base material 81 such as plastic, polypropylene laminate nonwoven fabric, nonwoven fabric and paper, and a base part of the tape base material 81 is joined to the side flap SF. A hook part 82 is provided on a surface, on the skin side (i.e., on the top sheet 2 side), of a protrusion piece of a tape base material 81. The hook part 82 is bonded to the tape base material 81 by an adhesive in a non-detachable manner, and has a plurality of engaging pieces. In the finished product, the connecting tape 8 is folded on the top sheet 2 side, with the hook part 82 detachably attached to the gather sheet 71.

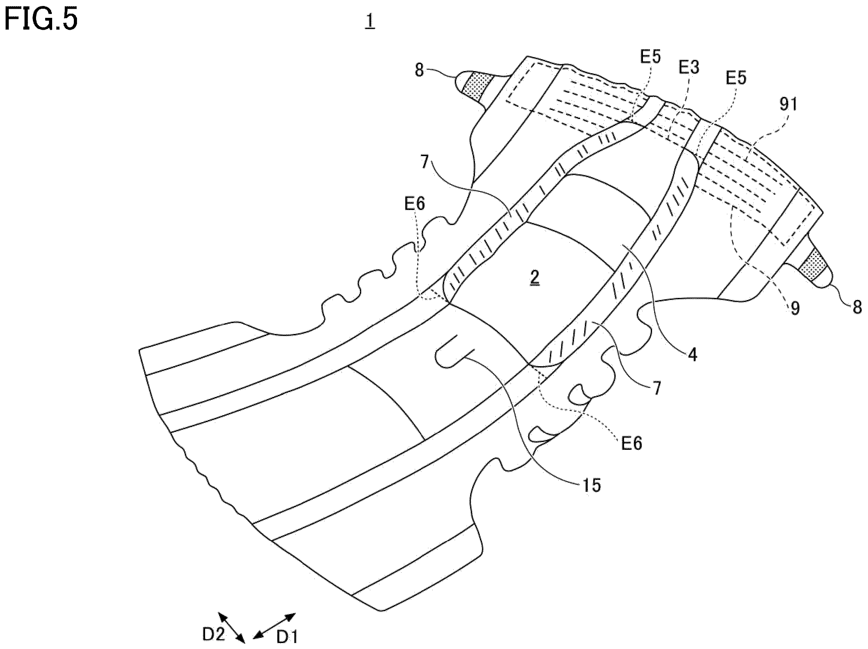

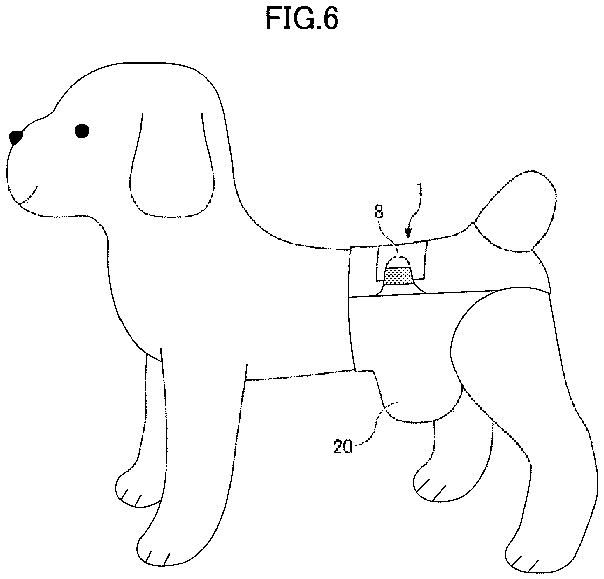

Next, the relationship between the absorber 4, the rising gather 7, and the waist sheet 9 will be described in detail. FIG. 5 is a perspective view of an absorbent article according to an embodiment, and FIG. 6 is a schematic view showing a state of an absorbent article according to an embodiment when worn on a pet.

A distance between a ventral-side edge E4 of the waist sheet 9 and a ventral-side edge E1 of the absorbent article 1 is 0 mm to 5 mm, a distance between a ventral-side edge E3 of the absorber 4 and a ventral-side edge E1 of the absorbent article 1 is 15 mm to 35 mm, and a distance between a fixation starting end E5 of the rising gather 7 on the ventral side and the ventral-side edge E3 of the absorber 4 is 0 mm to 5 mm. Here, the distance means the shortest distance in the front-back direction D1. As shown in FIG. 5, the rising gather 7 rises from a position within 5 mm from the ventral-side edge (front end) E3 of the absorber 4, and a contraction force of the rising gather 7 in the front-back direction D1 is exerted from the ventral-side end of the absorber 4 to the dorsal side. The waist sheet 9 includes a first region R1 and a second region R2. The first region R1 overlaps a portion of the absorber 4 on the ventral side, and the second region R2 extends beyond the first region R1 toward the ventral side and toward an outer side in the width direction D2, yet does not overlap the absorber 4. The second region R2 is elastically stretchable in the width direction D2, and the first region R1 is not elastically stretchable in the width direction D2 over the absorber 4. With this configuration, when a male pet wears the absorbent article 1, the second region R2 of the waist sheet 9 is stretchable elastically, so that the ends of the absorber 4 in the width direction D2 contract inwardly and come into close contact with the abdomen of the male pet. On the other hand, since the first region R1 overlapping the absorber 4 is not elastically stretchable over the absorber 4, the absorber 4 located in the first region R1 does not come into close contact with the abdomen of the male pet and becomes loose. At the same time, the rising gather 7 rises from a position within 5 mm from the ventral-side edge E3 of the absorber 4, and the contraction force of the rising gather 7 in the front-back direction D1 acts on the absorber 4 located in the first region R1. Then, as shown in FIG. 6, the absorber 4 forms a deformed protrusion part 20 that protrudes from the ventral-side end to the non-skin side, and then is separated from the male pet's abdomen. Thus, a urine capture space is formed in the absorbent article 1, in which the front and surrounding area of the male pet's urethral opening is surrounded by the absorber 4 with a space therebetween. Therefore, the absorbent article 1 can sufficiently capture urine with the absorber 4, thereby suppressing leakage of urine to the outside. Further, since the urethral opening and the absorber 4 are separated, the urine absorbed by the absorber can be prevented from contacting and adhering to the urethral opening.

Preferably, the distance between the fixation starting end E5 of the rising gather 7 on the ventral side and the ventral-side edge E3 of the absorber 4 is 0 mm. Here, the distance between the fixation starting end E5 of the rising gather 7 on the ventral side and the ventral-side edge E3 of the absorber 4 being 0 mm means that the distance between the fixation starting end E5 and the ventral-side edge (front end) E1 of the absorbent article 1 is the same as the distance between the ventral-side edge E3 of the absorber 4 and the ventral-side edge E1 of the absorbent article 1. As a result, when the male pet wears the absorbent article, the deformed protrusion part 20 which protrudes from the ventral-side edge E3 of the absorber 4 to the non-skin side is formed, so that the urine capture space is formed more forward and the absorber 4 is effectively utilized for capturing urine from the ventral-side edge E3. Therefore, the absorbent article can capture urine more sufficiently with the absorber, and thus can further suppress leakage of urine to the outside.

The length of the first region R1 of the waist sheet 9 in the front-back direction D1 is preferably equal to or less than the distance between the ventral-side edge of the second region R2 (the ventral-side edge E4 of the waist sheet 9) and the first region R1 (the ventral-side edge E3 of the absorber 4). Here, the distance means the shortest distance. As a result, the area of the second region of the waist sheet 9 which is closer to the ventral side than the first region R1 is sufficiently secured. Therefore, the ventral-side edge E1 of the absorbent article 1 can be brought into close contact with the pet's abdomen sufficiently, thereby further suppressing leakage of urine to the outside. In addition, since the area of the absorber 4 located in the first region R1 becomes smaller, the height of the protrusion part 20 of the absorber 4 becomes higher, and thus the urine capture space can be made larger, thereby further suppressing leakage of urine to the outside.

The first region R1 and the second region R2 of the waist sheet 9 may include an elastic member 91 which is stretchable in the width direction D2. Specifically, as shown in FIG. 4, the waist sheet 9 may include an elastic member 91 that is provided between the two nonwoven sheets 92, 92 and the two nonwoven sheets 92, 92 and that is stretchable in the width direction D2. In this case, the elastic member 91 in the first region R1 has been cut. In this way, by cutting the elastic member fixed in the first region R1 overlapping the absorber 4 using a shirring cutter, etc., after providing the elastic member in the first region R1 and the second region R2 of the waist sheet 9, the second region R2 thus is elastically stretchable in the width direction D2, while the first region R1 overlapping the absorber 4 is not elastically stretchable in the width direction D2 over the absorber 4. With this configuration, the absorber 4 forms a deformed protrusion part 20 which protrudes from the ventral-side end to the non-skin side, and a urine capture space is formed in the absorbent article 1, in which the front and surrounding area of the male pet's urethral opening is surrounded by the absorber 4 with a space therebetween. Therefore, the absorbent article 1 can sufficiently capture urine with the absorber, thereby suppressing leakage of urine to the outside.

In the waist sheet 9, the second region R2 may include an elastic member 91 that is elastically stretchable in the width direction D2, and the first region R1 may include, only in a portion of the dorsal-side end thereof, an elastic member 91 that is elastically stretchable in the width direction D2. By providing the elastic member 91 in the second region R2 of the waist sheet 9, the second region R2 is elastically stretchable in the width direction D2. Since the first region R1 that overlaps the absorber 4 includes an elastic member 91 only in a portion of the dorsal-side end, the first region R1 has a small contraction force, and thus can be of a structure that is not elastically stretchable in the width direction D2 over the absorber 4. Accordingly, the absorber 4 forms a deformed protrusion part 20 which protrudes from the ventral-side end to the non-skin side, and a urine capture space is formed in the absorbent article 1, in which the front and surrounding area of the male pet's urethral opening is surrounded by the absorber 4 with a space therebetween. Accordingly, the absorbent article 1 can sufficiently capture urine with the absorber 4, thereby suppressing leakage of urine to the outside.

The elastic member 91 may be a thread rubber, a stretchable film, or a member with a nonwoven fabric bonded thereto. As a material constituting the elastic member 91, a generally-used styrene-based rubber, an olefin-based rubber, a urethane-based rubber, an ester-based rubber, a polyurethane, a polyethylene, a polystyrene, a styrene-butadiene, a silicone, a polyester, or the like can be used.

The tension of the elastic member 91 is preferably 2. 0 cN to 10. 0 cN. Since the tension of the elastic member 91 is 2. 0 cN to 10. 0 cN, when the male pet wears the absorbent article 1, the height of the protrusion part 20 protruding toward the non-skin side can be made sufficiently high, and the absorber 4 can be sufficiently separated from the abdomen of the male pet. As a result, a urine capture space of sufficient size can be formed in the absorbent article 1, and the absorbent article 1 can further suppress leakage of urine to the outside.

In another embodiment, the waist sheet 9 may be configured such that the second region R2 includes an elastic member 91 that is elastically stretchable in the width direction D2, and the first region R1 does not include an elastic member 91. By providing the elastic member 91 only in the second region R2 of the waist sheet 9, the second region R2 can be elastically stretchable in the width direction D2, and the first region R1 overlapping the absorber 4 cannot be elastically stretchable over the absorber 4 in the width direction D2. With this configuration, the absorber 4 forms a deformed protrusion part 20 which protrudes from the ventral-side end to the non-skin side, and a urine capture space is formed in the absorbent article 1, in which the front and surrounding area of the male pet's urethral opening is surrounded by the absorber 4 with a space therebetween. Therefore, the absorbent article 1 can sufficiently capture urine with the absorber, thereby suppressing leakage of urine to the outside.

In yet another embodiment, the waist sheet 9 may be a sheet-like elastic member, such as a rubber sheet, which is elastically stretchable in the width direction D2 without the elastic member 91 included therein.

The distance between the fixation starting end E6 of the rising gather 7 on the dorsal side and the dorsal-side edge E2 of the absorbent article 1 is preferably 120 mm to 200 mm. Thus, when the absorbent article 1 is worn on a female pet, the rising gather 7 rises toward the skin side from the ventral side of the female pet to both sides of its urethral opening, so that the absorbent article 1 can suppress leakage of urine to the outside even when the absorbent article 1 is worn on the female pet. That is, the absorbent article 1 can suppress leakage of urine to the outside regardless of whether the male pet or the female pet wears the absorbent article 1.

The absorbent article 1 shown in FIG. 1 to FIG. 6 is illustrated as a fastening-type absorbent article 1 having a connecting tape 8, but the absorbent article 1 may be a pants-type absorbent article. In this case, both sides of the ventral-side region F and both sides of the dorsal-side region B are joined, respectively, and thus the absorbent article 1 has a waist opening for passing the pet's trunk and a pair of leg openings for passing its rear legs. The structure of each member can be similar to that of the absorbent article 1 shown in FIG. 1 to FIG. 6. Therefore, the absorbent article 1 can suppress leakage of urine to the outside even in the case of being a pants type.

### (Aspect of the present invention)

The present invention includes the following aspects.

### <Aspect 1>

An absorbent article for pets, including a front-back direction, a width direction orthogonal to the front-back direction, a dorsal-side region, and a ventral-side region,
wherein the absorbent article includes:
   a top sheet having liquid permeability,
   a waterproof sheet having liquid impermeability,
   an absorber provided between the top sheet and the waterproof sheet,
   a rising gather provided on both sides of the absorbent article in the width direction over the entire front-back direction, the rising gather rising toward a skin side, and
   a belt-shaped waist sheet extending in the width direction along a ventral-side edge of the absorbent article;
wherein a distance between a ventral-side edge of the waist sheet and the ventral-side edge of the absorbent article is 0 mm to 5 mm;
wherein a distance between a ventral-side edge of the absorber and the ventral-side edge of the absorbent article is 15 mm to 35 mm;
wherein the rising gather has both ends thereof in the front-back direction fixed to the top sheet;
wherein a distance between a fixation starting end of the rising gather on a ventral side thereof and the ventral-side edge of the absorber is 0 mm to 5 mm;
wherein the waist sheet includes:
   a first region overlapping a portion of the absorber on a ventral side thereof, and
   a second region extending beyond the first region toward the ventral side and toward an outer side in the width direction, the second region not overlapping the absorber; and
   wherein the second region is elastically stretchable in the width direction, and the first region is not elastically stretchable in the width direction over the absorber.

According to the above aspect 1, when a male pet wears the absorbent article, the second region of the waist sheet is stretchable elastically, so that the ends of the absorber in the width direction contracts inwardly and comes into close contact with the abdomen of the male pet. On the other hand, since the first region overlapping the absorber is not elastically stretchable over the absorber, the absorber located in the first region does not come into close contact with the abdomen of the male pet and becomes loose. At the same time, the rising gather rises from a position within 5 mm from the ventral-side edge of the absorber, and the contraction force of the rising gather in the front-back direction acts on the absorber located in the first region. As a result, the absorbent forms a deformed protrusion part which protrudes from the ventral-side edge to the non-skin side, and is separated from the abdomen of the male pet. Thereby, a urine capture space is formed in the absorbent article, in which the front and surrounding area of the male pet's urethral opening is surrounded by the absorber with a space therebetween. Therefore, the absorbent article can sufficiently capture urine with the absorber, thereby suppressing leakage of urine to the outside. Moreover, since the urethral opening and the absorber are separated, the urine absorbed by the absorber can be prevented from contacting and adhering to the urethral opening.

### <Aspect 2>

The absorbent article for pets according to aspect 1, wherein a length of the first region in the front-back direction is equal to or less than a distance between the first region and the ventral-side edge of the second region.

According to the above aspect 2, since the area of the second region of the waist sheet which is closer to the ventral side than the first region is sufficiently secured, the ventral-side end of the absorbent article can be brought into close contact with the pet's abdomen sufficiently, thereby further suppressing leakage of urine to the outside. In addition, since the area of the absorber located in the first region becomes smaller, the height of the protrusion part of the absorber becomes higher, and thus the urine capture space can be made larger, thereby further suppressing leakage of urine to the outside.

### <Aspect 3>

The absorbent article for pets according to aspect 1 or 2, wherein the first region and the second region each has an elastic member that is elastically stretchable in the width direction, and wherein the elastic member in the first region has been cut.

According to the above aspect 3, by cutting the elastic member fixed in the first region overlapping the absorber, after providing the elastic member in the first region and the second region of the waist sheet, the second region thus is elastically stretchable in the width direction, while the first region overlapping the absorber is not elastically stretchable in the width direction over the absorber. Therefore, the absorber forms a deformed protrusion part which protrudes from the ventral-side end to the non-skin side, and a urine capture space is formed in the absorbent article, in which the front and surrounding area of the male pet's urethral opening is surrounded by the absorber with a space therebetween. Therefore, the absorbent article can sufficiently capture urine with the absorber, thereby suppressing leakage of urine to the outside.

### <Aspect 4>

The absorbent article for pets according to aspect 1 or 2, wherein the second region has an elastic member that is elastically stretchable in the width direction, and the first region does not have an elastic member.

According to the above aspect 4, by providing the elastic member only in the second region of the waist sheet, the second region can be elastically stretchable in the width direction, and the first region overlapping the absorber cannot be elastically stretchable in the width direction over the absorber. Accordingly, the absorber forms a deformed protrusion part which protrudes from the ventral-side end to the non-skin side, and a urine capture space is formed in the absorbent article, in which the front and surrounding area of the male pet's urethral opening is surrounded by the absorber with a space therebetween. Therefore, the absorbent article can sufficiently capture urine with the absorber, thereby suppressing leakage of urine to the outside.

### <Aspect 5>

The absorbent article for pets according to aspect 1 or 2, wherein the second region has an elastic member that is elastically stretchable in the width direction, and the first region has, only in a portion of a dorsal-side end thereof, an elastic member that is elastically stretchable in the width direction.

According to the above aspect 5, by providing the elastic member in the second region of the waist sheet, the second region can be elastically stretchable in the width direction, and the first region overlapping the absorber includes an elastic member only in a portion of the dorsal-side end thereof, so that the contraction force is small and cannot be elastically stretchable in the width direction over the absorber. Accordingly, the absorber forms a deformed protrusion that protrudes from the ventral-side end to the non-skin side, and a urine capture space is formed in the absorbent article, in which the front and surrounding area of the male pet's urethral opening is surrounded by the absorber with a space therebetween. Therefore, the absorbent article can sufficiently capture urine with the absorber, thereby suppressing leakage of urine to the outside.

### <Aspect 6>

The absorbent article for pets according to any one of aspects 1 to 5, wherein a distance between a fixation starting end of the rising gather on a dorsal side thereof and a dorsal-side edge of the absorbent article is 120 mm to 200 mm.

According to the above aspect 6, when the absorbent article is worn on a female pet, the rising gather rises toward the skin side from the ventral side of the female pet to both sides of its urethral opening, so that the absorbent article can suppress leakage of urine to the outside even when the absorbent article is worn on the female pet.

As described above, the embodiments are presented as examples, and the present invention is not limited by the above embodiments. The embodiments can be implemented in various other forms, and various combinations, omissions, substitutions, changes, etc. can be made without departing from the gist of the invention. The above embodiments and their variations are included within the scope and essence of the invention, and are also included within the invention described in the claims and the equivalents thereof.

This international application claims priority under Japanese Patent Application No. 2022-154755 filed on September 28, 2022, the entire contents of which are incorporated herein by reference.

### DESCRIPTION OF SYMBOLS

1 absorbent article
2 top sheet
3 waterproof sheet
4 absorber
5 encapsulating sheet
6 back sheet
7 rising gather
71 gather sheet
8 connecting tape
81 tape base material
82 hook part
9 waist sheet
91 elastic member
92 non-woven sheet
10 second sheet
11 front target tape
12 leg-opening elastic member
13 gather elastic member
14a, 14b adhesive layer
15 cut part
20 protrusion part
SF side flap
EF end flap
F ventral-side region
B dorsal-side region
E1 ventral-side edge of the absorbent article
E2 dorsal-side edge of the absorbent article
E3 ventral-side edge of the absorber
E4 ventral-side edge of the waist sheet
E5 fixation starting end of the rising gather on the ventral side
E6 fixation starting end of the rising gather on the dorsal side
R1 first region
R2 second region
D1 front-back direction
D2 width direction

## Claims

1. An absorbent article for pets, the absorbent article comprising a front-back direction, a width direction orthogonal to the front-back direction, a dorsal-side region, and a ventral-side region,
wherein the absorbent article comprises:
a top sheet having liquid permeability,
a waterproof sheet having liquid impermeability,
an absorber provided between the top sheet and the waterproof sheet,
a rising gather provided on both sides of the absorbent article in the width direction over the entire front-back direction, the rising gather rising toward a skin side, and
a belt-shaped waist sheet extending in the width direction along a ventral-side edge of the absorbent article;
wherein a distance between a ventral-side edge of the waist sheet and the ventral-side edge of the absorbent article is 0 mm to 5 mm;
wherein a distance between a ventral-side edge of the absorber and the ventral-side edge of the absorbent article is 15 mm to 35 mm;
wherein the rising gather has both ends thereof in the front-back direction fixed to the top sheet;
wherein a distance between a fixation starting end of the rising gather on a ventral side thereof and the ventral-side edge of the absorber is 0 mm to 5 mm;
wherein the waist sheet includes:
a first region overlapping a portion of the absorber on a ventral side thereof, and
a second region extending beyond the first region toward the ventral side and toward an outer side in the width direction, the second region not overlapping the absorber; and
wherein the second region is elastically stretchable in the width direction, and the first region is not elastically stretchable in the width direction.

2. The absorbent article for pets according to claim 1, wherein a length of the first region in the front-back direction is equal to or less than a distance between the first region and a ventral-side edge of the second region.

3. The absorbent article for pets according to claim 2, wherein the first region and the second region each has an elastic member that is elastically stretchable in the width direction, and
wherein the elastic member in the first region has been cut.

4. The absorbent article for pets according to claim 2, wherein the second region has an elastic member that is elastically stretchable in the width direction, and the first region does not have an elastic member.

5. The absorbent article for pets according to claim 2, wherein the second region has an elastic member that is elastically stretchable in the width direction, and the first region has, only in a portion of a dorsal-side end thereof, an elastic member that is elastically stretchable in the width direction.

6. The absorbent article for pets according to any one of claims 3 to 5, wherein a distance between a fixation starting end of the rising gather on a dorsal side thereof and a dorsal-side edge of the absorbent article is 120 mm to 200 mm.
